# EUROPEAN PATENT SPECIFICATION

(11) **EP 2 090 176 B1**
(45) Date of publication and mention of the grant of the patent: **24.04.2019**
(21) Application number: 09152656.6
(22) Date of filing: 12.02.2009
(51) Int. Cl.: A23D 7/00, A23D 9/00, A61K 31/20, A61P 3/10, A23D 9/013, A61K 31/201

(54) **Postprandial hyperglycemia-improving agent**
Wirkstoff zur Verbesserung der postprandialen Hyperglykämie
Agent d'amélioration d'hyperglycémie postprandiale

(30) Priority: 13.02.2008 JP 2008031832; 06.01.2009 JP 2009000781
(43) Date of publication of application: 19.08.2009
(73) Proprietor: Kao Corporation, Chuo-Ku Tokyo 103-8210 (JP)
(72) Inventor: Osaki, Noriko, Haga-gun Tochigi 321-3497 (JP); Onizawa, Koji, Haga-gun Tochigi 321-3497 (JP); Shimotoyodome, Akira, Haga-gun Tochigi 321-3497 (JP)
(74) Representative: Hoffmann Eitle

(56) References cited:
- EP-A- 1 407 674
- WO-A-2007/046441
- US-A1- 2003 198 727
- US-A1- 2004 062 847
- US-A1- 2005 123 667
- MORITA O ET AL: "Effects of dietary diacylglycerol oil on embryo/fetal development in rats" FOOD AND CHEMICAL TOXICOLOGY, PERGAMON, GB, vol. 46, no. 7, 1 July 2008 (2008-07-01), pages 2510-2516, XP022708014 ISSN: 0278-6915 [retrieved on 2008-04-12]
- TOMONOBU K ET AL: "Dietary diacylglycerol in a typical meal suppresses postprandial increases in serum lipid levels compared with dietary triacylglycerol", NUTRITION, ELSEVIER INC, US, vol. 22, no. 2, 1 February 2006 (2006-02-01), pages 128-135, XP027974108, ISSN: 0899-9007 [retrieved on 2006-02-01]
- TADA N ET AL: "Effects of diacylglycerol ingestion on postprandial hyperlipidemia in diabetes", CLINICA CHIMICA ACTA, ELSEVIER BV, AMSTERDAM, NL, vol. 353, no. 1-2, 1 March 2005 (2005-03-01), pages 87-94, XP027648960, ISSN: 0009-8981 [retrieved on 2005-03-01]
- Hidekatsu Yanai ET AL: "Effects of Diacylglycerol on Glucose, Lipid Metabolism, and Plasma Serotonin Levels in Lean Japanese", Obesity, vol. 16, no. 1, 1 January 2008 (2008-01-01), pages 47-51, XP055100413, ISSN: 1930-7381, DOI: 10.1038/oby.2007.46
- TAGUCHI H ET AL: "Double-blind controlled study on the effects of dietary diacylglycerol on postprandial serum and chylomicron triacylglycerol responses in healthy humans.", JOURNAL OF THE AMERICAN COLLEGE OF NUTRITION 2000 NOV-DEC, vol. 19, no. 6, November 2000 (2000-11), pages 789-796, XP009175952, ISSN: 0731-5724
- MORI Y ET AL: "Dietary diacylglycerol reduces postprandial hyperlipidemia and ameliorates glucose intolerance in Otsuka Long-Evans Tokushima Fatty (OLETF) rats", NUTRITION, ELSEVIER INC, US, vol. 21, no. 9, 1 September 2005 (2005-09-01), pages 933-939, XP027768645, ISSN: 0899-9007 [retrieved on 2005-09-01]
- J. Tuomilehto: "Point: A Glucose Tolerance Test Is Important for Clinical Practice", DIABETES CARE, vol. 25, no. 10, 1 October 2002 (2002-10-01), pages 1880-1882, XP055247783, US ISSN: 0149-5992, DOI: 10.2337/diacare.25.10.1880
- J. J. MEIER ET AL: "Excess glycaemic excursions after an oral glucose tolerance test compared with a mixed meal challenge and self-measured home glucose profiles: is the OGTT a valid predictor of postprandial hyperglycaemia and vice versa?", DIABETES, OBESITY AND METABOLISM, vol. 11, no. 3, 1 March 2009 (2009-03-01), pages 213-222, XP055248017, ISSN: 1462-8902, DOI: 10.1111/j.1463-1326.2008.00922.x

## Description

### Field of the Invention

The present invention relates to a postprandial hyperglycemia-improving agent, which is useful as a food or a pharmaceutical product.

### Background of the Invention

After meals, particularly containing carbohydrates, an increase of blood glucose level promotes secretion of insulin. Insulin promotes intake of carbohydrates in adipose tissues or muscles to store the carbohydrates as fats (in adipose cells) or glycogen (in muscle cells). In addition, insulin suppresses gluconeogenesis in the liver (release of glucose from the liver). As described above, insulin acts on the liver, muscle, adipose tissue, or the like to suppress an acute increase of the blood glucose level after a meal. However, when a high blood glucose level is maintained to continue the secretion of insulin, diminished insulin sensitivity (insulin resistance) is caused in insulin target organs such as muscles, liver, and adipose tissues, resulting in an increase of the insulin secretion from the pancreas. If the insulin secretion is repeated, the pancreas is finally exhausted to decrease the insulin secretion from pancreatic beta cells. However, the increased insulin resistance in the respective target organs is kept. It is known that, if the action mechanism of insulin does not function normally as described above, the person tends to develop easily obesity, diabetes, or the like, and further develops obesity, type II diabetes mellitus (hyperglycemia), or the like.

In recent years, there have been reported that postprandial hyperglycemia or hyperlipidemia is an independent risk factor in a cardiovascular event (Non-Patent Document 1 and Non-Patent Document 2), and that fasting hyperglycemia has a low correlation with the probability of death due to cardiovascular diseases, while persons with the 2 hour blood glucose levels of 200 mg/dL or more, which is measured by oral glucose tolerance test (OGTT), have a high correlation with the probability of death due to cardiovascular diseases (Non-Patent Document 2). Moreover, it has also been reported that, when vascular endothelial cells are cultured in a hyperglycemic state, cell apoptosis is more frequently caused in culture in an intermittent hyperglycemic state compared with culture in a continuous hyperglycemic state (Non-Patent Document 3).

Therefore, suppression of the acute increase of the blood glucose level after a meal and improvement of hyperinsulinism caused by the increase of the blood glucose level is considered to lead to prevention of diabetes and arteriosclerosis.

Compared with a healthy subject, type I diabetes mellitus is characterized by the impaired insulin secretion and type II diabetes mellitus is characterized by the defective responsiveness of body tissues to insulin. The postprandial blood glucose level in the healthy subject is controlled by insulin, and the level does not exceed 7.8 mmol/L (140 mg/dL) after a meal and usually returns to the level before a meal within 2 or 3 hours (Non-Patent Document 4 and Non-Patent Document 5). However, in type I and type II diabetes mellitus having diminished insulin functions, postprandial hyperglycemia is frequently observed. Moreover, the postprandial hyperglycemia is a phenomenon caused before clinically obvious diabetic symptoms such as progressive diminishing of insulin functions or beta-cell functions (decreasing of insulin secretion), so that prevention of the postprandial hyperglycemia is considered to lead to prevention of diabetes and arteriosclerosis.

Based on the above-mentioned standpoint, administration of an α-glucosidase inhibitor for delaying digestion/absorption of carbohydrates in the small intestine or administration of a sulfonium urea-based preparation or rapid-acting insulin secretion promoter for promoting secretion of insulin are known to be an effective method to improve the postprandial hyperglycemia.

However, the α-glucosidase inhibitor has problems in that the inhibitor affects only by administration before ingestion of carbohydrates, has no effect on an increase in a blood glucose level caused by ingestion of glucose as monosaccharide, and causes intestine symptoms such as diarrhea and gas accumulation due to abnormal fermentation of carbohydrates in the colon. The sulfonium urea-based preparation should be used with discretion, because the preparation affects only by administration before ingestion of carbohydrates or may cause excessive secretion of insulin when administered excessively, resulting in hypoglycemia. Such medical synthetic preparations are not easily available because it requires prescriptions, and administration of the preparations may cause numerous side-effects. Before use of the preparations, tight control/guidance of a doctor is required.

Therefore, a postprandial hyperglycemia-improving agent, which can be routinely used and has few side effects, has been demanded.

On the other hand, diglycerides have an effect of suppressing an increase of a blood triglyceride level after a meal (Patent Document 1 and Non-Patent Document 6) and an effect of increasing a blood HDL cholesterol level after a meal (Patent Document 2), and hence they are used in cooking oils and various fat and oil-processed foods. Meanwhile, it is known that long-term ingestion of a diglyceride including an ω3 unsaturated fatty acid such as eicosapentaenoic acid, docosahexaenoic acid, or α-linolenic acid as a constituent fatty acid to obesity/diabetes model animals has the effect of decreasing blood glucose levels (Patent Document 3) .

As for the relationship between the diglyceride and postprandial hyperglycemia, it is known that a single ingestion of a meal, which contains a diglyceride in which the ω3 unsaturated fatty acid content in constituent fatty acids is less than 15% by mass and the oleic acid content in constituent fatty acids is 39.41%, can decrease the insulin secretion but cannot decrease the blood glucose level in a healthy subject (Patent Document 4). However, there is no report on the relationship between the diglyceride and postprandial hyperglycemia in diabetes patients having decreased insulin functions.
[Patent Document 1] JP-A-2003-2835
[Patent Document 2] JP-A-2001-64170
[Patent Document 3] JP-A-2001-247457
[Patent Document 4] JP-A-2007-45789
[Non-Patent Document 1] Diabetes Care. 1999; 22: 920-924
[Non-Patent Document 2] Arch Intern Med. 2001; 161: 397-405
[Non-Patent Document 3] Am J Physiol Endocrinol Metab. 2001; 281: E924-930
[Non-Patent Document 4] Diabetes Care. 2001; 24 (4): 775-778
[Non-Patent Document 5] J Clin Invest. 1988; 81 (2) : 442-448
[Non-Patent Document 6] J Am Coll Nutr. 2000; 19 (6) : 789-796

WO 2007046441 relates to a pet food comprising (A) an oil-and-fat and (B) at least one substance selected from modified starch, barley, sorghum, corn and high-amylose starch as a hydrocarbon source and containing a diacylglucerol in an amount of 20% by weight or more relative to the total amount of the oil-and-fat.

US 2003198727 (A1) concerns an oil/fat composition comprising 10.1 to 94.9 wt. % of a triglyceride, 0.1 to 30 wt. % of a monoglyceride and 5 to 59.9 wt. % of a diglyceride which has, as a fatty acid constituent thereof, 15 to 90 wt. % of an omega3-unsaturated fatty acid having less than 20 carbon atoms.

EP 1407674 concerns oil or fat compositions comprising: (A) an oil or fat containing from 60 to 100% by weight of diglyceride, by 100 parts by weight of the oil or fat composition, the diglyceride further comprising fatty acids, wherein the amount of fatty acids that are unsaturated is from 80 to 100 wt. %, by 100 parts by weight of the diglyceride; (B) from 0.001 to 1% by weight of ingredient (A) of a carboxylic acid selected from C2-8 hydroxycarboxylic acids, dicarboxylic acids and tricarboxylic acids, and salts derivatives thereof, and mixtures thereof; (C) from 0.001 to 5% by weight of ingredient (A) of an antioxidant; and (D) from 0.05 to 4.7% by weight of ingredient (A) of a plant sterol.

Hidekatsu Yanai et al., Obesity 16(1), 47-51 (2008) refer to a study in healthy test persons, examining the effects of diacyl glycerol on glucose and lipid metabolism.

Taguchi H. et al., J. Am. Coll. Nutrition 19(6), 789-796 (2000) concerns a double-blind controlled study on the effect of dietary diacyl glycerols in healthy test persons.

K. Tomonobu et al., Nutrition 22, 128-135 (2006) describe a study in healthy test persons, comparing the effects of diacyl glycerides and triacyl glycerides.

N. Tada et al., Clin. Chim. Acta 353, 87-94 (2005) examine the effect of diacyl glycerol ingestion in diabetes patients.

S. Meguro et al., Lipids 41(4), 347-355 (2006) examines the effects of diacyl glycerol in comparison to triacyl glycerol in rats, for the cases of a normal diet and a high sucrose diet.

Mori Y. et al., Nutrition 21(9), 933-939 (2005) describe comparative tests in OLETF rats, which are a model system for Type II diabetes. The test involves a comparison between diacyl glycerol and triacyl gylcerol.

S. Saito et al., Metab. Clin. Exp. 56, 1566-1575 (2007) examine the effect of dietary diacyl glycerol on the development of diet-induced obesity and insulin resistance.

### Summary of the invention

The present invention relates to a fat and oil composition containing 50% to 95% by mass of a diglyceride, in which the ω9 unsaturated fatty acid content in constituent fatty acids is 35% by mass or more and the ω6 unsaturated fatty acid content is 2 to 60% by mass, for use in a method of suppressing a temporary increase of postprandial glucose levels caused by ingestion of a carbohydrate-containing meal, by ingestion of the composition during said meal, said meal having a carbohydrate content such that the calories of carbohydrates are 10 to 90% of the total calories thereof, in a person who has abnormal glucose tolerance or impaired insulin secretion.

### Brief description of the drawings

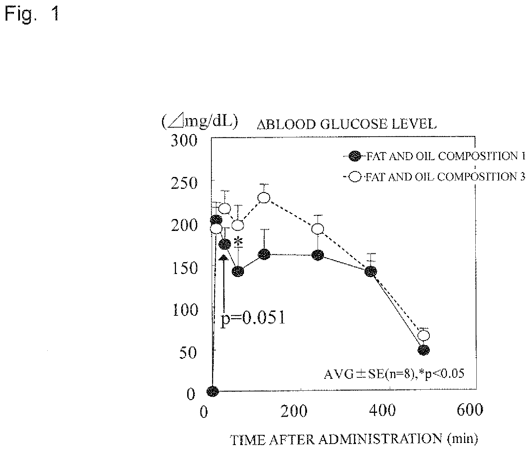

Fig. 1 is a graph illustrating time-dependent changes in a blood glucose level of each rat group administered with an emulsion containing glucose and the fat and oil composition 1 or 3 up to 480 minutes after administration.

### Detailed Description of the Invention

The present invention relates to a fat and oil composition for use in a method of suppressing a temporary increase of postprandial glucose levels caused by ingestion of a carbohydrate-containing meal, as defined above. The fat and oil composition is useful as a food or a pharmaceutical product.

The inventors of the present invention have made studies on a material for controlling a postprandial blood glucose level, and have found out that a fat and oil composition, which contains a diglyceride in which the ω9 unsaturated fatty acid content in constituent fatty acids is high, can effectively decrease an increased postprandial blood glucose level, and that a diglyceride having a specific fatty acid composition has the effect of improving postprandial hyperglycemia in a diabetes patient whose insulin function to an increased blood glucose level is relatively decreased. That is, the fat and oil composition, which contains a diglyceride in which the ω9 unsaturated fatty acid content in constituent fatty acids is high, is useful as a food or a pharmaceutical product for preventing or improving diabetes.

With the postprandial hyperglycemia-improving agent of the present invention, it is possible to suppress the postprandial blood glucose level within an appropriate range. agent can lead potential diabetes patients to prevention of the onset and to improvement of their body constitution.

In the present invention, the term "ω9 unsaturated fatty acid" refers to an unsaturated fatty acid in which the first unsaturated bond is located at the 9th carbon atom from the ω position when the positions of carbon-carbon unsaturated bonds are located based on the ω position (farthest from the carboxyl group) . The number of the carbon atoms in the ω9 unsaturated fatty acid is not particularly limited, but is preferably 8 to 24, and more preferably 16 to 22.

Preferred examples of the ω9 unsaturated fatty acid include oleic acid, eicosamonoenoic acid, docosamonoenoic acid, and erucic acid. Of those, oleic acid is more preferable.

The content of the ω9 unsaturated fatty acid contained as a constituent fatty acid in a diglyceride to be used in the postprandial hyperglycemia-improving agent of the present invention is 35% by mass or more, preferably 37% by mass or more, more preferably 40% by mass to 100% by mass, and still more preferably 50% by mass to 95% by mass. The content of erucic acid, which is an ω9 unsaturated fatty acid, is preferably 5% by mass or less, and more preferably 1% by mass or less.

If the fat and oil composition of the present invention containing the above-mentioned ω9 unsaturated fatty acid at a content of 35% by mass or more is administered to a person who has abnormal glucose tolerance or impaired insulin secretion (such as a type I diabetes mellitus or a type II diabetes mellitus), a good effect of improving postprandial hyperglycemia can be achieved. Therefore, use of the agent can lead potential diabetes patients to prevention of the onset and to improvement of their body constitution.

Examples of fatty acids other than the ω9 unsaturated fatty acid contained as a constituent fatty acid in a diglyceride include an ω3 unsaturated fatty acid, an ω6 unsaturated fatty acid, and a saturated fatty acid. The content of the ω3 unsaturated fatty acid contained as a constituent fatty acid is preferably 1% by mass to 14% by mass, more preferably 5% by mass to 14% by mass, still more preferably 7% by mass to 14% by mass, and even more preferably 8% by mass to 14% by mass. Meanwhile, the content of the ω6 unsaturated fatty acid contained as a constituent fatty acid is 2% by mass to 60% by mass. In addition, the content of the saturated fatty acids contained as a constituent fatty acid is preferably 30% by mass or less, more preferably 10% by mass or less, and still more preferably 5% by mass or less. Moreover, in order to enhance the effect of improving postprandial hyperglycemia, the ratio of cis-unsaturated + saturated) to (trans-unsaturated + saturated) is preferably 6 or more, more preferably 9 to 25, and still more preferably 9 to 20. The content of a trans-unsaturated fatty acid in the diglyceride is preferably 5% by mass or less.

The term "ω3 unsaturated fatty acid" refers to a fatty acid in which the first unsaturated bond is located at the 3rd carbon atom from the ω position when the positions of carbon-carbon unsaturated bonds are located based on the ω position, and which has 2 or more carbon-carbon unsaturated bonds . Of those, preferred is a fatty acid having 3 to 6 carbon-carbon unsaturated bonds. The number of the carbon atoms in the ω3 unsaturated fatty acid is not particularly limited, but is preferably 8 to 24, and more preferably 16 to 22. Preferred examples of ω3 unsaturated fatty acids having 20 or more carbon atoms include eicosapentaenoic acid and docosahexaenoic acid, while preferred examples of ω3 unsaturated fatty acids having less than 20 carbon atoms include α-linolenic acid.

The term "ω6 unsaturated fatty acid" refers to a fatty acid in which the first unsaturated bond is located at the 6th carbon atom from the ω position when the positions of carbon-carbon unsaturated bonds are located based on the ω position, and which has 2 or more carbon-carbon unsaturated bonds . Of those, preferred is a fatty acid having 2 to 6 carbon-carbon unsaturated bonds, and more preferred is a fatty acid having 2 to 4 carbon atoms. Preferred examples of the ω6 unsaturated fatty acid include linoleic acid, γ -linolenic acid, arachidonic acid, and the like. Of those, linoleic acid is preferable.

The amount of the unsaturated fatty acids in the constituent fatty acids is preferably 55% by mass or more, more preferably 70% by mass or more, and still more preferably 90% by mass or more with respect to the total constituent fatty acids.

The fat and oil composition of the present invention contains a diglyceride at a content of 50 to 95% by mass. From the viewpoint to achieve the effect of suppressing an increase of blood glucose levels, the content of diacylglyceride is preferably 55% by mass to 90% by mass.

From the viewpoint of flavor and oxidation stability, the content of the monoglyceride is preferably 0% by mass to 30% by mass, more preferably 0.1% by mass to 10% by mass, still more preferably 0.1% by mass to 5% by mass, even more preferably 0.1% by mass to 2% by mass, yet even more preferably 0.1% by mass to 1.5% by mass.

The fat and oil composition of the present invention may contain a triglyceride in addition to the diglyceride and monoglyceride. The content of the triglyceride is preferably 0.1% by mass to 50% by mass. The composition of fatty acids in the triglyceride is preferably the same as the fatty acid compositions of the diglyceride and monoglyceride.

In order to improve oxidation stability, the fat and oil composition may contain a glyceride polymer. The glyceride polymer is a product obtained by intermolecular polymerization of glycerides such as triglycerides, diglycerides, and monoglycerides (Chemistry and Biology, vol.21, pp.179, 1983), and the polymerization degrees of the glycerides and the positions of fatty acid esters are not particularly limited.

From the viewpoints of improvement of oxidation stability and flavor of the fat and oil composition, the content of the glyceride polymer in the fat and oil is preferably 0.1% by mass to 10% by mass, more preferably 0.2% by mass to 5% by mass, and still more preferably 0.3% by mass to 4% by mass. The amount of the glyceride polymer may be controlled by appropriately controlling reaction temperature conditions or the like during the glyceride synthesis and may be determined by HPLC with a gel filtration chromatography column. The content of free fatty acids in the fat and oil composition is preferably 5% by mass or less.

The fat and oil composition of the present invention may be produced by: fractionating triglycerides, diglycerides and monoglycerides obtained by a transesterification reaction between glycerin and a fat and oil composition containing ω9 unsaturated fatty acids such as rapeseed oil, olive oil and rice oil as constituent fatty acids; and appropriately mixing the resultant products. Alternatively, the composition may be produced by performing an esterification reaction catalyzed by lipase activity in a mixture of a given constituent fatty acid or an ester thereof and a glycerin. In addition to such production methods, the composition may be produced by separation of a natural edible fat and oil composition.

An ordinary vegetable oil contains a phytosterol at a content of approximately 0.05 to 1.2% by mass. A fat and oil composition containing a diglyceride may be produced by the above-mentioned methods, but such production methods may have decreased amount of the phytosterol. In such case, the phytosterol may be added so that the oil contains the phytosterol at 0.05% by mass or more. The upper limit of the content of the phytosterol is not particularly limited; however the content is preferably 0.3 to 2% by mass. Examples of the phytosterols include α-sitosterol, β-sitosterol, stigmasterol, campesterol, α-sitostanol, β-sitostanol, stigmastanol, campestanol, and fatty acid esters, ferulate esters and cinnamate esters thereof.

The fat and oil composition to be used in the postprandial hyperglycemia-improving agent of the present invention contains a diglyceride at a content of 50% by mass to 95% by mass.

As shown in Examples below, the fat and oil composition of the present invention, as defined above, can significantly suppress an increase of postprandial blood glucose levels both in diabetes model animals and normal animals, and the effect is much higher than that of a fat and oil composition containing a diglyceride and/or a monoglyceride in which the ω3 unsaturated fatty acid content is high (JP-A-2001-247457 above-mentioned). Therefore, the fat and oil composition of the present invention is useful for suppressing a temporary increase of postprandial glucose levels caused by ingestion of a carbohydrate-containing meal, by ingestion of the composition during said meal, said meal having a carbohydrate content such that the calories of carbohydrates are 10 to 90% of the total calories thereof, in a person who has abnormal glucose tolerance or impaired insulin secretion.

The postprandial hyperglycemia-improving agent can be used as a pharmaceutical product, quasi-drug or food for human or animals .

The postprandial hyperglycemia-improving agent of the present invention, which suppresses an increase of postprandial blood glucose levels, may be used as a cosmetic food, invalid food, or functional food such as a food for specified health use, indicating its effect if required.

The term "improvement of postprandial hyperglycemia" refers to suppression of a temporary increase of postprandial blood glucose levels, in particular that caused by ingestion of a meal containing carbohydrates.

Examples of an administration route of the postprandial hyperglycemia-improving agent of the present invention as a pharmaceutical product or a supplement include: oral administration with a tablet, capsule, granule, powder, or syrup; and parenteral administration with an injectable solution, suppository, inhalation agent, transdermal absorption agent, or external preparation. In order to prepare pharmaceutical preparations and supplements having such various dosage forms, the fat and oil of the present invention may be used singly or in an appropriate combination with another pharmaceutically acceptable vehicle, binder, extender, disintegrant, surfactant, lubricant, dispersant, buffer, preservative, flavoring agent, perfume, film-forming agent, carrier, or diluent. Among those administration routes, oral administration is preferable, and the content of the fat and oil of the present invention in an oral administration preparation is 0.1% by mass to 100% by mass, preferably 1% by mass to 100% by mass, and still more preferably 5% by mass to 100% by mass based on the whole composition.

Examples of the form in the case where the postprandial hyperglycemia-improving agent of the present invention is used as a food include various foods such as breads, cakes, noodles, snacks, jellies, frozen food products, ice creams, dairy products, drinks, mayonnaise-like foods such as mayonnaise and salad dressing, and the same forms as the above-mentioned oral administration preparations such as tablets, capsules, syrup, or the like.

To prepare foods in various forms, the fat and oil composition of the present invention is used singly, or in an appropriate combination with another food material, solvent, softener, oil, emulsifier, antiseptic, perfume, stabilizer, coloring agent, antioxidant, humectant, thickener, or the like. The content of the fat and oil of the present invention in the foods is 0.1% by mass to 90% by mass, preferably 1% by mass to 85% by mass, and more preferably 5% by mass to 80% by mass based on the whole composition.

In the case where the postprandial hyperglycemia-improving agent is used as a pharmaceutical product or a food, the administration or ingestion amount of the fat and oil of the present invention per adult per day is preferably 0.05 to 50 g, and more preferably 1 to 10 g. The postprandial hyperglycemia-improving agent of the present invention is effective when used during a meal.

Accordingly, the postprandial hyperglycemia-improving agent of the present invention is ingested during a meal. For this purpose, the whole or part of an ordinary used cooking oil may be replaced with the above-described fat and oil composition of the present invention. In this case, in order to improve postprandial hyperglycemia, the content of the fat and oil composition in fats in a meal is preferably 15% by mass or more, more preferably 40% by mass to 90% by mass, still more preferably 60% by mass to 95% by mass, even more preferably 70% by mass to 95% by mass, even more preferably 80% by mass to 95% by mass. Meanwhile, the postprandial hyperglycemia-improving agent of the present invention is ingested during a meal in which the calories of carbohydrates are 10 to 90%, preferably 15 to 80%, more preferably 20 to 70%, and even more preferably 30 to 60% based on the total calories of the meal. In addition, the postprandial hyperglycemia-improving agent of the present invention is preferably ingested during a meal so that the calories of the diglyceride are 5 to 60%, more preferably 10 to 50%, and still more preferably 15 to 45% based on the total calories of the meal. In the present invention, the number of calories is calculated as: 1 g of lipid=9 kcal; 1 g of carbohydrate=4 kcal; and 1 g of protein=4 kcal.

Examples of the meal using the postprandial hyperglycemia-improving agent of the present invention include heat cooked products such as deep-fried products, baked products, stir-fried products, boiled products, steamed products and cooked rice, and non-heat cooked products such as salad with dressing or mayonnaise and sandwiches.

Using the postprandial hyperglycemia-improving agent of the present invention, it is possible to provide a cooked product, which is not provided because of strict calorie restriction, or which is provided very little under a conventional alimentary therapy, resulting in an improvement of QOL.

### Examples

### Example 1

Diglycerides were produced by the following method.

The saturated fatty acid content in a fatty acid obtained by hydrolyzing a commercially-available rapeseed oil was reduced with wintering. Subsequently, an esterification reaction of the fatty acid and glycerin was performed at 40°C using a commercially-available lipase preparation (trade name: Lipozyme IM (NovoNordiskA/S)), which is an immobilized 1,3-position-specific lipase, as a catalyst. The lipase preparation was removed by filtration, and the resultant product was subjected to molecular distillation and purified, to thereby obtain diglycerides having the fatty acid compositions shown in Table 1.

Next, rapeseed oil, soybean oil, safflower oil, and perilla oil were mixed to prepare the triglycerides having the same fatty acid composition as those of the diglycerides shown in Table 1 (hereinafter, the term "% by mass" is abbreviated as "%").

**Table 1**

| (%) | | | | | | | | |
|---|---|---|---|---|---|---|---|---|
| | | | Fat and oil composition | | | | | |
| | | | Inventive product | | Comparative product | | | |
| | | | 1 | 2 | 3 | 4 | 5 | 6 |
| Glyceride composition | | TG | 12.8 | 14.6 | 97.3 | 98.7 | 98.1 | 5.4 |
| | | DG | 87.2 | 84.9 | 2.7 | 1.3 | 1.3 | 94.3 |
| | | MG | 0.0 | 0.5 | 0.0 | 0.0 | 0.0 | 0.2 |
| | | FFA | 0.1 | 0.1 | 0.0 | 0.0 | 0.0 | 0.1 |
| Constituent fatty acid | ω9 | C18:1 | 38.0 | 61.2 | 37.0 | 59.1 | 19.2 | 19.4 |
| | | C24:1 | 0.1 | 0.1 | 0.3 | 0.3 | 0.2 | 0.2 |
| | ω3 | C18:3 | 8.1 | 10.1 | 8.3 | 12.5 | 58.8 | 59.3 |
| | ω6 | C18:2 | 48.3 | 21.0 | 44.9 | 21.4 | 13.0 | 12.9 |
| | Saturated | C16:0 | 3.0 | 4.4 | 5.3 | 4.2 | 6.0 | 5.7 |
| | | C18:0 | 1.1 | 2.1 | 2.0 | 1.8 | 2.4 | 2.2 |
| | | C20:0 | 0.3 | 0.7 | 0.5 | 0.6 | 0.1 | 0.0 |
| | | C22:0 | 0.2 | 0.2 | 0.3 | 0.0 | 0.0 | 0.0 |
| | | C24:0 | 0.1 | 0.2 | 0.2 | 0.2 | 0.2 | 0.2 |
| | Monoene other than ω9 | C20:1 | 0.7 | 0.0 | 0.9 | 0.0 | 0.0 | 0.0 |
| | | C22:1 | 0.0 | 0.1 | 0.1 | 0.0 | 0.0 | 0.0 |

### Example 2 Suppressive effect of diglyceride on increase of postprandial blood glucose level in severe type I diabetes model rat

Six-week-old male SD rats were used to create severe type I diabetes model rats. Streptozotocin dissolved in citrate buffer (pH 4.5) was intraperitoneally administered to the SD rats fasted for 17 hours in an amount of 70 mg per kg body weight, and rats having casual blood glucose levels of 300 mg/dL or more (average: 431 mg/dL) after one week were defined as severe type I diabetes model rats. The severe type I diabetes model rats were divided into groups of ten rats each and fasted for 17 hours. Glucose (2 mg/g body weight) and the fat and oil composition 1 or 3 shown in Table 1 (2 mg/g body weight) were emulsified by egg lecithin (Wako Pure Chemicals Industries, Ltd., 0.04 mg/g body weight) and bovine serum albumin (Sigma, 0.4 mg/g body weight). Then the each emulsion was administered orally through a probe. Blood samples were collected via the tail veins with time until 60 minutes after administration, and blood glucose levels were measured using a portable blood-glucose level measuring device (Accu-Chek Aviva, Roche Diagnostics K.K.).

Table 2 shows the relative values of maximum increase of blood glucose levels (amount of the maximum increase of blood glucose level until 60 minutes after administration of the emulsion containing the glucose and the fat and oil composition 3 to the rats is defined as 100) and relative AUC values of increased blood glucose levels (AUC value of the increased blood glucose level until 60 minutes after administration of the emulsion containing the glucose and the fat and oil composition 3 to the rats is defined as 100).

**Table 2**

| | Amount of maximum increase of blood glucose level (Relative value AVG±SD) | AUC value of increased blood glucose level until 60 minutes after administration (Relative value AVG±SD) |
|---|---|---|
| Inventive product (fat and oil composition 1) | 56.5±23.9*** | 64.7±22.5** |
| Comparative product (fat and oil composition 3) | 100.0±23.9 | 100.0±21.2 |

| | | |
|---|---|---|
| Statistically significance of the fat and oil composition 1 with respect to the fat and oil composition 3 **p<0.01, ***p<0.001 | | |

The results of Table 2 reveal that ingestion of the fat and oil composition 1 together with glucose has the effect of suppressing the maximum increase of postprandial blood glucose levels and the amount of increase of postprandial blood glucose.

### Example 3 Suppressive effect of diglyceride on increase of postprandial blood glucose level in mild type I diabetes model rat

Six-week-old male SD rats were used to create mild type I diabetes model rats. Streptozotocin dissolved in citrate buffer (pH 4.5) was intraperitoneally administered to the SD rats in an amount of 55 mg per kg body weight, and rats having casual blood glucose levels of 190 mg/dL or more (average: 264 mg/dL) after one week were defined as mild type I diabetes model rats. The mild type I diabetes model rats were divided into groups of six rats each and fasted for 17 hours. Glucose (2 mg/g body weight) and the fat and oil composition 1 or 3 shown in Table 1 (2 mg/g body weight) were emulsified by egg lecithin (Wako Pure Chemicals Industries, Ltd., 0.04 mg/g body weight) and bovine serum albumin (Sigma, 0.4 mg/g body weight) . Then the each emulsion was administered orally through a probe. Blood samples were collected via the tail veins with time until 60 minutes after administration, and blood glucose levels were measured using a portable blood-glucose level measuring device (Accu-Chek Aviva, Roche Diagnostics K.K.).

Table 3 shows the relative values of maximum increase of blood glucose levels (amount of the maximum increase of blood glucose level until 60 minutes after administration of the emulsion containing the glucose and the fat and oil composition 3 to the rats is defined as 100) and relative AUC values of increased blood glucose levels (AUC value of the increased blood glucose level until 60 minutes after administration of the emulsion containing the glucose and the fat and oil composition 3 to the rats is defined as 100).

**Table 3**

| | Amount of maximum increase of blood glucose level (Relative value AVG±SD) | AUC value of increased blood glucose level until 60 minutes after administration (Relative value AVG±SD) |
|---|---|---|
| Inventive product (fat and oil composition 1) | 80.4±10.6* | 74.4±14.5* |
| Comparative product (fat and oil composition 3) | 100.0±17.1 | 100.0±17.7 |

| | | |
|---|---|---|
| Statistically significance of the fat and oil composition 1 with respect to the fat and oil composition 3 *p<0.05 | | |

The results of Table 3 reveal that ingestion of the fat and oil composition 1 together with glucose to the type I diabetes model rats has the effect of suppressing the maximum increase of postprandial blood glucose levels and the amount of increase of postprandial blood glucose, that is, the effect of decreasing the increased blood glucose levels.

### Example 4 Suppressive effect of diglyceride on increase of postprandial blood glucose level in type II diabetes model rat

Eight male ZDF-*Lepr^{fa}*/CrlCrlj rats at 11 weeks of age having casual blood glucose levels of 300 mg/dL or more (purchased from Charles River Laboratories Japan, Inc.) were used. After fasting for 17 hours, glucose (2 mg/g body weight) and the fat and oil composition 1 or 3 shown in Table 1 (2 mg/g body weight) were emulsified by egg lecithin (Wako Pure Chemicals Industries, Ltd., 0.04 mg/g body weight) and bovine serum albumin (Sigma, 0.4 mg/g body weight) . Then the each emulsion was administered orally through a probe. Blood samples were collected via the tail veins with time until 480 minutes after administration, and blood glucose levels were measured using a portable blood-glucose level measuring device (Accu-Chek Aviva, Roche Diagnostics K.K.). Moreover, 10 days after completion of the first test, recovery of the body weights and casual blood glucose levels was confirmed, and the second test was performed using another emulsion (cross-over test).

Fig. 1 shows time-dependent changes in the blood glucose levels of each rat group administered with the emulsion containing the glucose and the fat and oil composition 1 or 3 until 480 minutes, and Table 4 shows the relative AUC values of increased blood glucose levels (AUC value of the increased blood glucose level until 120 minutes after administration of the emulsion containing the glucose and the fat and oil composition 3 to the rats is defined as 100).

**Table 4**

| | AUC value of increased blood glucose level until 120 minutes after administration (Relative value AVG±SD) |
|---|---|
| Inventive product (fat and oil composition 1) | 77.9±34.4* |
| Comparative product (fat and oil composition 3) | 100.0±22.4 |

| | |
|---|---|
| Statistically significance of the fat and oil composition 1 with respect to the fat and oil composition 3 *p<0.05 | |

The results of Fig. 1 and Table 4 reveal that ingestion of the fat and oil composition 1 together with glucose to the type II diabetes model rats has the effect of suppressing the increase of postprandial blood glucose levels and the amount of increase of postprandial blood glucose, that is, the effect of decreasing the increased blood glucose levels.

### Example 5 Suppressive effect of diglyceride on increase of postprandial blood glucose level in normal mouse (Reference Example)

Eight-week-old male C57BL/6J mice fasted for 17 hours were allowed ad libitum access to the diets described in Table 5 for one hour to ingest the diets. Blood samples were collected via the fundus veins at 1, 2, and 5 hours after the ingestion, and blood glucose levels were measured using a portable blood-glucose level measuring device (Accu-Chek Aviva, Roche Diagnostics K.K.). Table 5 shows the amounts of the diets ingested by mice ad libitum for one hour, relative values of maximum increase of blood glucose levels (amount of the maximum increase of blood glucose level until 5 hours after ingestion of the diet containing the fat and oil composition 4 is defined as 100), and relative AUC values of increased blood glucose levels (AUC value of the increased blood glucose level until 5 hours after ingestion of the diet containing the fat and oil composition 4 is defined as 100).

**Table 5**

| (AVG±SD) | | | | | |
|---|---|---|---|---|---|
| | | Inventive product (fat and oil composition 2) (n=8) | Comparative product (fat and oil composition 4) (n=7) | Comparative product (fat and oil composition 5) (n=8) | Comparative product (fat and oil composition 6) (n=10) |
| Diet composition (%) | Inventive product (fat and oil composition 2) | 30.0 | - | - | - |
| | Comparative product (fat and oil composition 4) | - | 30.0 | - | - |
| | Comparative product (fat and oil composition 5) | - | - | 30.0 | - |
| | Comparative product (fat and oil composition 6) | - | - | - | 30.0 |
| | sucrose | 13.0 | 13.0 | 13.0 | 13.0 |
| | Casein | 20.0 | 20.0 | 20.0 | 20.0 |
| | Cellulose | 4.0 | 4.0 | 4.0 | 4.0 |
| | Starch | 28.5 | 28.5 | 28.5 | 28.5 |
| | Mineral mixture | 3.5 | 3.5 | 3.5 | 3.5 |
| | Vitamin mixture | 1.0 | 1.0 | 1.0 | 1.0 |
| One-hour ingestion amount (g) | | 0.68±0.05 | 0.72±0.05 | 0.72±0.05 | 0.68±0.05 |
| Amount of maximum increase of blood glucose level (Relative value) | | 67.9±18.6* | 100.0±24.8 | 95.6±28.4 | 79.9±13.7 |
| AUC value of increased blood glucose level until 5 hours after ingestion (Relative value) | | 78.0±15.1* | 100.0±18.4 | 90.6±21.4 | 82.3±12.9 |

| | | | | | |
|---|---|---|---|---|---|
| Statistically significance of the fat and oil composition 2 with respect to the fat and oil composition 4 *p<0.05 | | | | | |

The results of Table 5 reveal that ingestion a diet of the fat and oil composition 2 having an oleic acid content of 60% by mass or more to the normal mice has the effect of suppressing the maximum increase of postprandial blood glucose levels and the amount of increase of postprandial blood glucose, that is, the effect of decreasing the increased blood glucose levels. The fat and oil composition 6, which has been reported to have the effect of decreasing blood glucose levels in long-term administration (JP-A-2001-247457 above-mentioned), was found to have lower effect of improving postprandial hyperglycemia compared with the product of the present invention.

### Reference Example

Eight-week-old male C57BL/6J mice fasted for 17 hours were divided into groups of 11 or 12 mice each. Glucose and lipid (triolein) (2 mg/g body weight each) was emulsified by egg lecithin (Wako Pure Chemicals Industries, Ltd., 0.02 mg/g body weight). Then glucose (2 mg/g body weight) only or the emulsion was administered orally through a probe. Blood samples were collected via the fundus veins with time until 60 minutes after administration, and blood glucose levels were measured using a portable blood-glucose level measuring device (Accu-Chek Aviva, Roche Diagnostics K.K.). Table 6 shows the relative values of maximum increase of blood glucose levels (amount of the maximum increase of blood glucose level in mice administered with glucose only is defined as 100).

**Table 6**

| | Amount of maximum increase of blood glucose level (Relative value AVG±SD) |
|---|---|
| Glucose only | 100.0±8.5 |
| Glucose + triolein | 94.2±13.8 |

As shown in Table 6, there is no difference in the amount of the maximum increase of blood glucose level between administration of glucose only and administration of the emulsion containing the glucose and the lipid (triolein).

## Claims

1. A fat and oil composition containing 50% to 95% by mass of a diglyceride, in which the ω9 unsaturated fatty acid content in constituent fatty acids is 35% by mass or more and the ω6 unsaturated fatty acid content is 2 to 60% by mass,
for use in a method of suppressing a temporary increase of postprandial glucose levels caused by ingestion of a carbohydrate-containing meal, by ingestion of the composition during said meal, said meal having a carbohydrate content such that the calories of carbohydrates are 10 to 90% of the total calories thereof,
in a person who has abnormal glucose tolerance or impaired insulin secretion.

## Patentansprüche

1. Fett- und Ölzusammensetzung, enthaltend 50 bis 95 Massen-% eines Diglycerids, wobei der Gehalt an ω9-ungesättigten Fettsäuren in den Fettsäurebestandteilen 35 Massen-% oder mehr beträgt und der Gehalt an ω6-ungesättigten Fettsäuren 2 bis 60 Massen-% beträgt,
zur Verwendung in einem Verfahren zur Unterdrückung eines vorübergehenden Anstiegs des postprandialen Glucosespiegels, hervorgerufen durch die Einnahme einer kohlenhydrathaltigen Mahlzeit, durch Einnahme der Zusammensetzung während der Mahlzeit, wobei die Mahlzeit einen solchen Kohlenhydratgehalt aufweist, dass die Kalorien der Kohlenhydrate 10 bis 90% von dessen Gesamtkalorien ausmachen,
bei einer Person mit abnormaler Glukosetoleranz oder gestörter Insulinsekretion.

## Revendications

1. Composition de graisse et d'huile contenant 50% à 95% en masse d'un diglycéride, dans laquelle la teneur en acides gras insaturés ω9 dans des acides gras constitutifs est de 35 % en masse ou plus et la teneur en acides gras insaturés ω6 est de 2 à 60 % en masse,
pour une utilisation dans un procédé de suppression d'une augmentation temporaire de taux de glucose post-prandiale provoquée par l'ingestion d'un repas contenant des glucides, par l'ingestion de la composition lors dudit repas, ledit repas ayant une teneur en glucides telle que les calories des glucides représentent 10 à 90% des calories totales de celui-ci,
chez une personne qui présente une tolérance anormale au glucose ou une sécrétion altérée d'insuline.
